# EUROPÄISCHE PATENTANMELDUNG

(11) **EP 2 149 556 A1**
(43) Veröffentlichungstag der Anmeldung: **03.02.2010**
(21) Anmeldenummer: 08013724.3
(22) Anmeldetag: 31.07.2008
(51) Int. Cl.: C07D 213/20

(54) **Verfahren zur Herstellung von unsymmetrisch substituierten Dipyrazolyloxyphenyl-Derivaten**

(71) Anmelder: Bayer CropScience Aktiengesellschaft, 40789 Monheim (DE)
(72) Erfinder: Ford, Mark, 61389 Oberreifenberg (DE)

(57) **Zusammenfassung**

Es wird ein Verfahren zur Herstellung von unsymmetrisch substituierten Dipyrazolyloxyphenyl-Derivaten durch basenkatalysierte Reaktion eines symmetrisch substituierten Dipyrazolyloxyphenyl-Derivats mit einem Hydroxypyrazol beschrieben.

## Beschreibung

Die Erfindung betrifft das technische Gebiet der Pflanzenschutzmittel.

Spezieller betrifft sie ein Verfahren zur Herstellung von unsymmetrisch substituierten Dipyrazolyloxyphenyl-Derivaten.

Dipyrazolyloxyphenyl-Derivate der allgemeinen Formel (I) sind als herbizid wirksame Verbindungen beispielsweise aus WO 03/51846 bekannt.

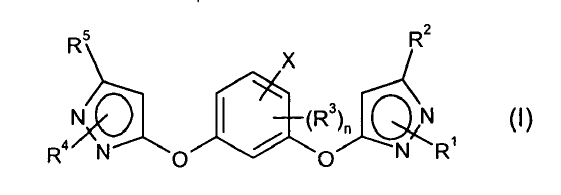

Aus WO 03/51846 ist auch bekannt, dass die Herstellung von unsymmetrisch substituierten Dipyrazolyloxyphenyl-Derivaten, d.h. für den Fall, dass die beiden Pyrazolreste nicht gleichartig substituiert sind, im Gegensatz zu den symmetrisch substituierten Derivaten, nicht immer in zufriedenstellenden Ausbeuten und Reinheiten gelingt.

Dem Fachmann beispielsweise aus WO 03/51846 bekannte Verfahren zur Herstellung von unsymmetrisch substituierten Dipyrazolyloxyphenyl-Derivaten (II) beruhen auf einer basenkatalysierten Austauschreaktion eines Hydroxyprazols (IV) gegen beispielsweise ein Halogenatom eines Pyrazolyloxyphenyl-Derivats (III) gemäß Schema 1.

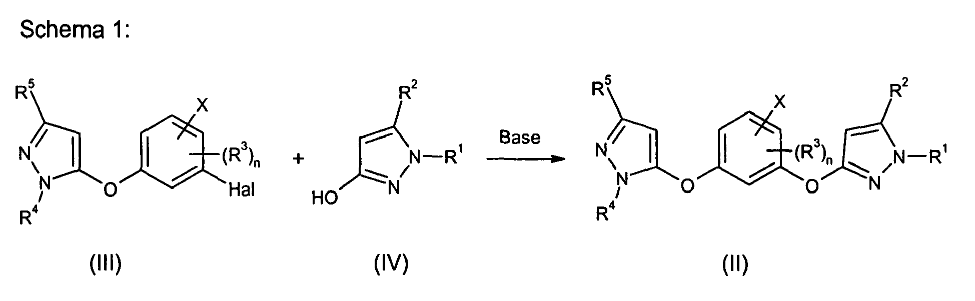

Dabei tritt jedoch unter anderem als Konkurrenzreaktion gleichzeitig der Austausch des Hydroxyprazols (IV) gegen den bereits in der Verbindung (III) gebundenen Pyrazolyloxy-Rest gemäß Schema 2 auf, so dass schwierig zu trennende Substanzgemische entstehen, und die gewünschten Derivate (II) nur in geringen Ausbeuten anfallen.

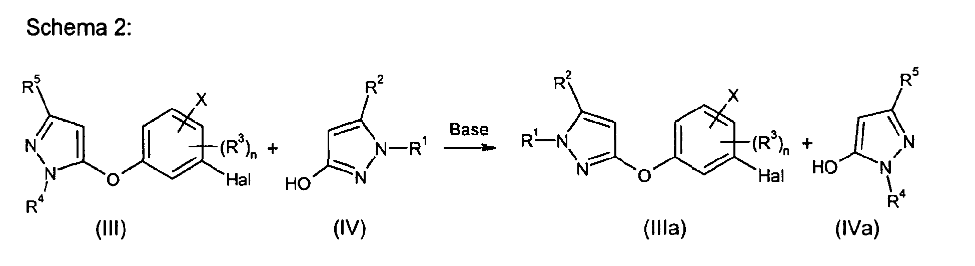

Aufgabe der vorliegenden Erfindung war die Bereitstellung eines Verfahrens, das die Herstellung von unsymmetrisch substituierten Dipyrazolyloxyphenyl-Derivaten in hohen Ausbeuten und Reinheiten ermöglicht. Es wurde gefunden, dass Reaktionsbedingungen die basenkatalysierte Reaktion eines Hydroxyprazols mit einem symmetrisch substituierten Dipyrazolyloxyphenyl-Derivaten zu unsymmetrisch substituierten Dipyrazolyloxyphenyl-Derivaten in hohen Ausbeuten und Reinheiten führt.

Ein Gegenstand der vorliegenden Erfindung ist somit ein Verfahren zur Herstellung von unsymmetrisch substituierten Dipyrazolyloxyphenyl-Derivaten der Formel (II), 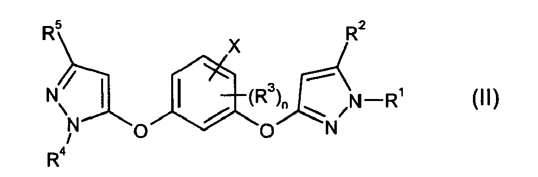 worin
- R¹: (C₁-C₈)-Alkyl, Benzyl oder durch 1, 2 oder 3 gleiche oder verschiedene Reste aus der Gruppe Methyl, Methoxy und Chlor substitutiertes Benzyl bedeutet;
- R²: Trifluormethyl, Difluormethyl oder Chlordifluoromethyl bedeutet;
- R³: Fluor, Chlor, Brom, lod, Methyl, Ethyl, Methoxy, Methylthio oder Trifluormethyl bedeutet;
- R⁴: (C₁-C₈)-Alkyl, Benzyl, durch 1, 2 oder 3 gleiche oder verschiedene Reste aus der Gruppe Methyl, Methoxy und Chlor substitutiertes Benzyl oder Trifluor-ethyl bedeutet;
- R⁵: Fluormethyl, Difluormethyl, Trifluormethyl, Fluormethoxy, Difluormethoxy, Trifluormethoxy, Pentafluorethyl, Chlor oder Cyano bedeutet;
- X: Nitro, Cyano, Trifluormethyl oder Methylsulfonyl bedeutet, und
- n: 0, 1 oder 2 bedeutet,
und das dadurch gekennzeichnet ist, dass ein symmetrisch substituiertes Dipyrazolyloxyphenyl-Derivat der Formel (IIa), worin die Symbole dieselbe Bedeutungen haben wir für Formel (II) definiert, 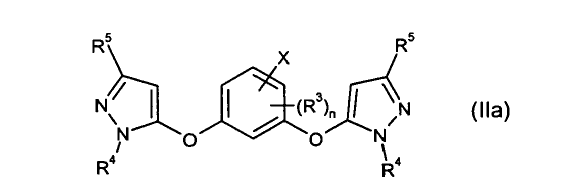 mit einem Hydroxyprazol der Formel (IV), worin die Symbole dieselbe Bedeutungen haben wir für Formel (II) definiert, 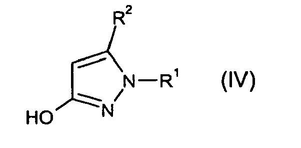 unter Basenkatalyse umgesetzt wird.

Die hier verwendeten Verbindungen der Formel (IIa) sind beispielsweise nach den in WO 03/51846 angegebenen Methoden zugänglich.

In den hier genannten Definitionen können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl.

Geeignete Basen sind tertiäre und aromatische Amine, wie Triethylamin, Picolin, 3-Alkyllyridin, N,N-Dimethylaminopyridin, N-Alkylimidazol, Benzotriazol, Pyridin, Alkali-und Erdalkalimetallcarbonate, wie Natrium-, Kalium-, Magnesium- und Calciumcarbonat, Alkalimetallphosphate, wie Natrium- und Kaliumphosphat, Alkalimetallalkoxide, wie Natriummethoxid, Natriumethoxid, Natrium-t-butoxid, Kaliummethoxid, Kaliumethoxid und Kalium-t-butoxid, Alkali- und Erdalkalimetallhydroxide, wie Natriumhydroxid, Kaliumhydroxid und Calciumhydroxid.

Das Hydroxyprazol der Formel (IV) wird üblicherweise in einer Menge von 1 bis 5, vorzugsweise 1 bis 3, besonders bevorzugt 1 bis 1,5 Moläquivalenten, bezogen auf die Menge des Dipyrazolyloxyphenyl-Derivats der Formel (IIa), eingesetzt.

Üblicherweise wird die Base in einer Menge von 0,01 bis 5, vorzugsweise 0,01 bis 2 und besonders bevorzugt 0,01 bis 1,2 Moläquivalenten, bezogen auf die Menge des Hydroxyprazols der Formel (IV), eingesetzt.

In einer alternativen Ausführungsform wird in einem der Austauschreaktion vorgelagerten Schritt das Hydroxyprazol der Formel (IV) mit einer der oben genannten Basen in das zugehörige Salz überführt und dieses dann mit dem Dipyrazolyloxyphenyl-Derivat der Formel (IIa) umgesetzt.

In einer bevorzugten Ausführungsform werden Dipyrazolyloxyphenyl-Derivate der Formel (IIa) verwendet, bei denen der Rest X sich in ortho-Position zur auszutauschenden Pyrazolyloxygruppe befindet.

Während der Reaktion können Alkohol und/oder Wasser gebildet werden. Alkohol und/oder Wasser werden vorzugsweise durch geeignete Hilfsmittel, wie Molekularsieb, oder azeotrop entfernt.

Die Reaktion wird üblicherweise in einem inerten Lösungsmittel, beispielsweise solchen aus der Gruppe der Ether und Polyethern, wie Dioxan, Tetrahydrofuran, Methyltetrahydrofuran, Dimethoxyethan, Diethylenglykoldimethylether oder Gemischen daraus durchgeführt.

Die Reaktion wird üblicherweise bei einer Temperatur von 40°C bis zum Siedepunkt des verwendeten Lösungsmittels, vorzugsweise zwischen 50 und 150°C, besonders bevorzugt zwischen 50 und 100°C durchgeführt. Die Reaktion kann auch unter Druck durchgeführt werden um die erforderliche Reaktionstemperatur durch Erhöhen des Siedepunkts des Lösungsmittels zu erreichen. Üblicherweise wird sie bei einem Druck zwischen 0,5 und 6 bar durchgeführt.

Die Reaktion kann vorzugsweise in Anwesenheit eines Ammonium- oder Phosphonium-Phasentransfer-Katalysators, wie Tetrabutylammoniumbromid und Tetraphenyphosphoniumbromid durchgeführt werden.

Das erfindungsgemäße Verfahren zeichnet sich auch dadurch aus, dass die bei der Reaktion anfallenden Nebenprodukte, wie das vom Dipyrazolyloxyphenyl-Derivat der Formel (IIa) ausgetauschte Hydroxypyrazol, sehr leicht abgetrennt werden kann, wie durch Abfiltrieren oder durch Herauswaschen mit basisch gestelltem Wasser.

Für den Fall, dass n Null bedeutet, sind die Verbindungen der Formel (II) neu. Ein weiterer Gegenstand der vorliegenden Erfindung sind somit Verbindungen der Formel (IIb), worin die Symbole dieselbe Bedeutungen haben wir für Formel (II) definiert.

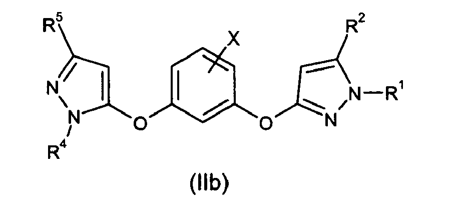

Verbindungen der Formel (IIb) sind ebenfalls als herbizid wirksame Verbindungen von Interesse.

### Ausführungsbeispiel

### Herstellung von 2-(1-Methyl-5-trifluormethylpyrazol-3-yloxy)- 4-(1-methyl-3-trifluormethylpyrazol-5-yloxy)-nitrobenzol

10,0 g 2,4-Di-(1-Methyl-3-trifluoromethylpyrazol-5-yloxy)-nitrobenzol, 4,78 g 3-Hydroxy-1-methyl-5-trifluoromethylpyrazol und 3,98 g Kaliumcarbonat werden in 40ml Methyltetrahydrofuran suspendiert und auf 80°C erhitzt. Nach 14 Stunden läßt man auf Raumtemperatur abkühlen und filtriert ab. Das Filtrat wird im Vakuum eingeengt, und der Rückstand wird mit Toluol verdünnt und dann mit verdünnter Kaliumhydroxid-Lösung und Wasser gewaschen. Nach dem Trocknen über Natiumsulfat wird im Vakuum eingeengt. Man erhält 9,8 g 2-(1-Methyl-5-trifluormethylpyrazol-3-yloxy)- 4-(1-methyl-3-trifluormethylpyrazol-5-yloxy)-nitrobenzol als Feststoff (Ausbeute 98% der Theorie).

## Patentansprüche

1. Verfahren zur Herstellung von unsymmetrisch substituierten Dipyrazolyloxyphenyl-Derivaten der Formel (II), 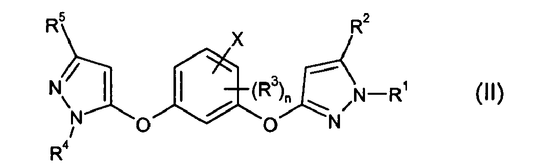 worin
R¹ (C₁-C₈)-Alkyl, Benzyl oder durch 1, 2 oder 3 gleiche oder verschiedene Reste aus der Gruppe Methyl, Methoxy und Chlor substitutiertes Benzyl bedeutet;
R² Trifluormethyl, Difluormethyl oder Chlordifluoromethyl bedeutet;
R³ Fluor, Chlor, Brom, lod, Methyl, Ethyl, Methoxy, Methylthio oder Trifluormethyl bedeutet;
R⁴ (C₁-C₈)-Alkyl, Benzyl, durch 1, 2 oder 3 gleiche oder verschiedene Reste aus der Gruppe Methyl, Methoxy und Chlor substitutiertes Benzyl oder Trifluorethyl bedeutet;
R⁵ Fluormethyl, Difluormethyl, Trifluormethyl, Fluormethoxy, Difluormethoxy, Trifluormethoxy, Pentafluorethyl, Chlor oder Cyano bedeutet;
X Nitro, Cyano, Trifluormethyl oder Methylsulfonyl bedeutet,
und
n 0, 1 oder 2 bedeutet,
und das **dadurch gekennzeichnet ist, dass** ein symmetrisch substituiertes Dipyrazolyloxyphenyl-Derivat der Formel (IIa), worin die Symbole dieselbe Bedeutungen haben wir für Formel (II) definiert, 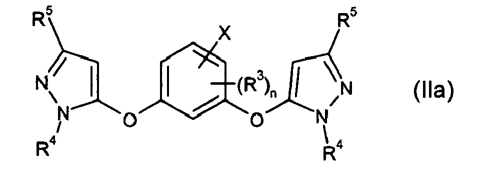 mit einem Hydroxyprazol der Formel (IV), worin die Symbole dieselbe Bedeutungen haben wir für Formel (II) definiert, 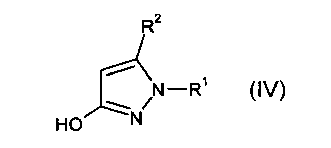 unter Basenkatalyse umgesetzt wird.

2. Verfahren nach Anspruch 1, **dadurch gekennzeichnet, dass** das Hydroxyprazol der Formel (IV) in einer Menge von 1 bis 3 Moläquivalenten, bezogen auf die Menge des Dipyrazolyloxyphenyl-Derivats der Formel (IIa), eingesetzt wird.

3. Verfahren nach Anspruch 1 oder 2, **dadurch gekennzeichnet, dass** als Base eine aus der Gruppe tertiäre und aromatische Amine, Alkali- und Erdalkalimetallcarbonate, Alkalimetallphosphate, Alkalimetallalkoxide, Alkali- und Erdalkalimetallhydroxide verwendet wird.

4. Verfahren nach einem der Ansprüche 1 bis 3, **dadurch gekennzeichnet, dass** die Base in einer Menge von 0,01 bis 2 Moläquivalenten, bezogen auf die Menge des Hydroxyprazols der Formel (IV), eingesetzt wird.

5. Verfahren nach einem der Ansprüche 1 bis 4, **dadurch gekennzeichnet, dass** die Reaktion in einem inerten Lösungsmittel durchgeführt wird.

6. Verfahren nach Anspruch 5, **dadurch gekennzeichnet, dass** das Lösungsmittel aus der Gruppe der Ether und Polyether stammt.

7. Verfahren nach einem der Ansprüche 1 bis 6, **dadurch gekennzeichnet, dass** es bei einer Temperatur von 50 bis 100°C durchgeführt wird.

8. Verfahren nach einem der Ansprüche 1 bis 7, **dadurch gekennzeichnet, dass** es in Anwesenheit eines Ammonium- oder Phosphonium-Phasentransfer-Katalysators durchgeführt wird.

9. Verfahren nach einem der Ansprüche 1 bis 8, **dadurch gekennzeichnet, dass** Dipyrazolyloxyphenyl-Derivate der Formel (IIa) verwendet werden, bei denen der Rest X sich in ortho-Position zur auszutauschenden Pyrazolyloxygruppe befindet.

10. Verbindungen der Formel (IIa), 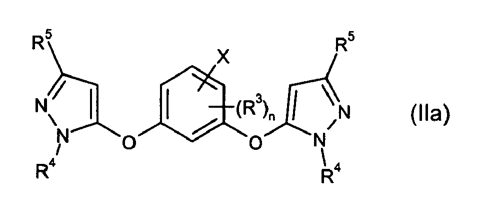 worin die Symbole dieselbe Bedeutungen haben wir für Formel (II) definiert.
